# EUROPÄISCHE PATENTSCHRIFT

(11) **EP 1 824 595 B1**
(45) Veröffentlichungstag und Bekanntmachung des Hinweises auf die Patenterteilung: **18.08.2010**
(21) Anmeldenummer: 05813888.4
(22) Anmeldetag: 24.11.2005
(51) Int. Cl.: B01J 23/30, B01J 23/04, B01J 27/132, C07C 319/04

(54) **HALOGENIDHALTIGE CAESIUMWOLFRAMATE ENTHALTENDE KATALYSATOREN ZUR SYNTHESE VON ALKYLMERCAPTAN UND VERFAHREN ZU IHRER HERSTELLUNG**
CATALYSTS COMPRISING HALIDE-CONTAINING CESIUM TUNGSTATES FOR SYNTHESIZING ALKYL MERCAPTANE, AND METHOD FOR THE PRODUCTION THEREOF
CATALYSEURS CONTENANT DES TUNGSTATES DE CESIUM CONTENANT DES HALOGENURES, DESTINES A LA SYNTHESE DE MERCAPTANS D'ALKYLES ET PROCEDES DE FABRICATION

(30) Priorität: 18.12.2004 DE 102004061016
(43) Veröffentlichungstag der Anmeldung: 29.08.2007
(73) Patentinhaber: Evonik Degussa GmbH, 45128 Essen (DE)
(72) Erfinder: REDLINGSHÖFER, Hubert, 91481 Münchsteinach (DE); WECKBECKER, Christoph, 63584 Gründau-Lieblos (DE); DÖRFLEIN, Andreas, 63538 Grosskrotzenburg (DE)
(86) Internationale Anmeldenummer: PCT/EP2005/012598
(87) Internationale Veröffentlichungsnummer: WO 2006/063669

(56) Entgegenhaltungen:
- EP-A- 0 832 687
- EP-A- 0 832 878
- WO-A-2004/096760
- WO-A-2005/021491
- WO-A-2006/015668
- MASHKINA A V ET AL: "ACTIVITY OF TUNGSTATE CATALYSTS IN THE SYNTHESIS OF METHYL - MERCAPTANE FROM METHANOL AND HYDROGEN SULFIDE" REACTION KINETICS AND CATALYSIS LETTERS, ELSEVIER, AMSTERDAM, NL, Bd. 36, Nr. 1, 1988, Seiten 159-164, XP002063476 ISSN: 0133-1736

## Beschreibung

Die vorliegende Erfindung betrifft einen halogenidhaltige Cäsium wolframate enthaltenden Katalysator zur Synthese von Alkylmercaptanen aus Alkanolen und Schwefelwasserstoff.

Unter dem Begriff Halogenide sind die gebundene Halogene des Periodensystems der Elemente zu verstehen, wobei die erfindungsgemäßen Cäsium wolframate auch zwei oder mehr verschiedene Halogenide enthalten können.

Unter den Alkylmercaptanen ist insbesondere Methylmercaptan ein industriell wichtiges Zwischenprodukt zum Beispiel für die Synthese von Methionin sowie für die Synthese von Dimethylsulfoxid und Dimethylsulfon. Es wird heute überwiegend aus Methanol und Schwefelwasserstoff durch Reaktion an einem Katalysator aus Aluminiumoxid hergestellt. Die Synthese des Methylmercaptans erfolgt gewöhnlich in der Gasphase bei Temperaturen zwischen 300 und 500°C und bei Drücken zwischen 1 und 25 bar.

Das Reaktionsgemisch enthält neben dem gebildeten Methylmercaptan die nicht umgesetzten Ausgangsstoffe und Nebenprodukte, wie zum Beispiel Dimethylsulfid und Dimethylether sowie die im Sinne der Reaktion inerten Gase, wie zum Beispiel Methan, Kohlenmonoxid, Wasserstoff und Stickstoff. Aus diesem Reaktionsgemisch wird das gebildete Methylmercaptan abgetrennt.

Für die.Wirtschaftlichkeit des Verfahrens wird eine möglichst hohe Ausbeute bei der katalytischen Reaktion von Methanol und Schwefelwasserstoff zu Methylmercaptan gefordert, um den Aufwand bei der Abtrennung des gebildeten Methylmercaptans vom Reaktionsgemisch so gering wie möglich zu halten. Hier stellt insbesondere der Energieaufwand zur Kühlung des Reaktionsgasgemisches zur Kondensation des Methylmercaptans einen großen Kostenfaktor dar.

Zur Erhöhung von Aktivität und Selektivität wird Aluminiumoxid als Träger üblicherweise mit Kaliumwolframat oder Cäsiumwolframat versetzt. Dabei wird das Wolframat gewöhnlich in Mengen bis 25 Gew.-%, bezogen auf das Gesamtgewicht des Katalysators, eingesetzt. Eine Verbesserung von Aktivität und Selektivität erhält man auch durch Erhöhung des Molverhältnisses von Schwefelwasserstoff zu Methanol. Üblicherweise werden Molverhältnisse zwischen 1 und 10 angewendet.

Ein hohes Molverhältnis bedeutet allerdings auch einen hohen Überschuss des Schwefelwasserstoffes im Reaktionsgemisch und somit die Notwendigkeit, große Gasmengen im Kreis zu führen. Zur Verminderung des hierfür benötigten Energieaufwandes sollte daher das Verhältnis von Schwefelwasserstoff zu Methanol nur wenig von 1 abweichen.

Die US-PS 2,820,062 betrifft ein Verfahren zur Herstellung von organischen Thiolen, bei welchem ein Katalysator aus aktivem Aluminiumoxid verwendet wird, der mit Kaliumwolframat in einer Menge von 1,5 bis 15 Gew.-%, bezogen auf das Gewicht des Katalysators, versetzt ist. Mit diesem Katalysator werden gute Aktivitäten und Selektivitäten bei Reaktionstemperaturen von 400°C und Molverhältnissen von 2 erzielt. Diese US-Patentschrift nennt verschiedene Möglichkeiten zur Einbringung des Kaliumwolframats in das Aluminiumoxid. So sollen Imprägnierverfahren, Copräzipitationen und reine Mischungen anwendbar sein. Der eigentlichen Herstellung des Katalysators wird wenig Bedeutung für die Wirtschaftlichkeit des Syntheseverfahrens von Methylmercaptan eingeräumt.

In der EP 0 832 687 B1 werden die Vorteile der Verwendung von Cäsiumwolframat (Cs₂WO₄) anstelle von Kaliumwolframat (K₂WO₄) als Promotor beschrieben. So kann durch Einsatz von Cäsiumwolframat eine gesteigerte Aktivität bei gleichzeitig guter Selektivität erreicht werden.

Durch Erhöhung der Cäsiumwolframat-Konzentration auf bis zu 40 Gew.-% kann die Selektivität zu Methylmercaptan auf bis zu 92 % gesteigert werden, ohne dass die Aktivität unverhältnismäßig verschlechtert wird.

Nach allgemeiner Ansicht erzielt man die beste Selektivität mit Katalysatoren, bei denen das Alkali/Wolframverhältnis gleich 2:1 ist (A.V. Mashkina et al., React. Kinet. Catal. Lett., Vol. 36, No. 1, 159-164 (1988).

Aufgabe der vorliegenden Erfindung ist es, einen Katalysator zur verfügung zu stellens, welcher sich bei niedrigen Molverhältnissen von Schwefelwasserstoff zu Methanol durch verbesserte Aktivität und Selektivität gegenüber den bekannten Katalysatoren auszeichnet und somit zu einer besseren Ausbeute und höherer Wirtschaftlichkeit des Verfahrens führt.

Diese Aufgabe wird durch die Bereitstellung eines Katalysators gelöst, enthaltend ein katalytisch aktives Cäsium wolframat, welches gebundenes Cäsium und Wolfram mit einem molaren Verhältnis von Cäsium zu Wolfram vor < 4:1, insbesondere von 3:1 bis 0,9:1, bevorzugt 2,4:1 bis 1:1, besonders 2,2:1 bis 1,2:1 sowie Halogenid(e), insbesondere Halogenide und Cäsium mit einem molaren Verhältnis von 0,01:1 bis 3:1, bevorzugt 0,01:1 bis 1:1, besonders 0,1:1 bis 1:1, enthält.

Die erfindungsgemässe katalytisch aktive halogenidhaltige Verbindung hat im allgemeinen die Formel

A_{X}WO_{Y}X_{Z}, (I)

in der bedeuten,
A: Cs,
X: Halogenid(e), ausgewählt aus der Gruppe F, C1, Br, J
x: 0,9 bis < 4, insbesondere 1,2 bis 3;
y: dieser Wert stellt sich entsprechend der Struktur des Wolframats und des Cäsium gehalts aufgrund der 6-Wertigkeit des Wolframs ein;
z: 0,01 bis < 12, insbesondere 0,9 bis <4.

Die Größe von z ist ein Maß für den Halogenidgehalt im Wolframat, der nicht chemisch an das Wolframat gebunden vorliegen muss.

Der Halogenidbestandteil der Zusammensetzung gemäss Formel I besteht aus oder enthält insbesondere dann Chlorid, wenn das Wolframat mindestens ein weiteres Halogenid, ausgewählt aus der Gruppe F, Br, I, enthält.

Der gebundene Halogenbestandteil des Katalysators kann sich aus einem oder mehreren verschiedenen Halogeniden zusammensetzen.

Liegt der Katalysator als Trägerkatalysator vor, enthält er das halogenidhaltige Cäsium wolframat in einer Menge von 8 bis 50 Gew.-%, insbesondere 15 bis 40 Gew.-%, bevorzugt 20 bis 36 Gew.-%. Im Falle eines Schalenkatalysators beziehen sich diese Anteile auf die Zusammensetzung der Schale.

Die halogenhaltigen oxidischen Verbindungen aus Cäsium und Wolfram können direkt auf einen Trägerkörper imprägniert werden (Trägerkatalysator).

Bei der Herstellung von Katalysatoren in Form von Extrudaten oder Preßlingen wird der pulverförmige Träger mit der erfindungsgemäßen oxidischen Zusammensetzung imprägniert oder vermischt und die erhaltene Zwischenstufe anschließend verformt (Vollkatalysator). Wird ein Schalenkatalysator hergestellt, so wird der pulverförmigen Träger mit der katalytisch wirksamen Zusammensetzung imprägniert und die entstehende Mischung dann auf einen bevorzugt inerten Trägerkern in Form einer Schale aufgebracht.

Das molare Halogenid/Cäsium-Verhältnis beläuft sich besonders bevorzugt auf 0,1:1 bis 1:1. Damit enthalten die erfindungsgemäßen Wolframate für die Umsetzung von Alkanolen mit Schwefelwasserstoff zu Alkylmercaptanen im Unterschied zu den mit Cäsiumwolframat (Cs₂WO₄) oder Kaliumwolframat (K₂WO₄) imprägnierten Katalysatoren gemäss Stand der Technik einen Anteil an Halogeniden.

Es zeigt sich, dass der Anteil an Halogeniden insbesondere auf dem bevorzugt eingesetzten Aluminiumoxid im Vergleich zu dem im Stand der Technik ausschließlich verwendeten nicht halogenidfreien Alkaliwolframat dem Katalysator eine deutlich verbesserte Aktivität bei gleichzeitig hoher Selektivität verleiht. Ferner zeigt sich durch den Zusatz von Halogeniden zum Cäsium wolframat in unerwarteter Weise eine ausgezeichnete Selektivität bei sehr hohen Umsatzgraden an Alkohol. Erfindungsgemäß kann bei sehr hohen Beladungen mit dem Promotor ein ausgezeichneter Umsatz erreicht werden, ohne dass die Selektivität des Katalysators abnimmt, wie es aus dem Stand der Technik für halogenidfreien Katalysatoren bekannt ist. Weiterhin wurde gefunden, dass über das Cäsium-Wolfram-Halogenid-Verhältnis und über die Wahl der Halogenide die Aktivität und Selektivität des Katalysators gezielt eingestellt werden kann. Durch die Möglichkeit, Mischungen verschiedener Halogene einzusetzen, können vergleichsweise teure Substanzen wie Brom- oder Jod-Verbindungen zumindest teilweise durch kostengünstigere Chloride ersetzt werden, ohne dass die Aktivität oder Selektivität des Katalysators beeinträchtigt wird.

Der Katalysator wird bevorzugt in Form eines Trägerkatalysators, bei dem die Oberfläche mit der katalytisch wirksamen Substanz imprägniert wird, oder eines Schalenkatalysators, bei dem ein bevorzugt inerter Kern mit einem Gemisch aus katalytisch aktiver Substanz und Trägermaterial umgeben ist, eingesetzt. Weiterhin können Extrudate oder Preßlinge, bei denen die katalytisch aktive Substanz mit dem pulverförmigen Trägermaterial vor der Verformung vermischt bzw. diese mit ihr imprägniert wird, verwendet werden.

Als Trägermaterialien werden die bekannten oxidischen anorganischen Verbindungen wie zum Beispiel SiO₂, TiO₂, ZrO₂ und bevorzugt sogenanntes aktives Aluminiumoxid eingesetzt. Dieses Material weist hohe spezifische Oberflächen zwischen etwa 10 und 400 m²/g auf und besteht hauptsächlich aus Oxiden der Übergangsreihe der kristallographischen Phasen des Aluminiumoxids (siehe zum Beispiel Ullmann's Enzyclopedia of Industrial Chemistry von 1985, Vol. A1, Seiten 561-562). Zu diesen Übergangsoxiden gehören γ-, δ-, η-, κ-, χ- und θ-Aluminiumoxid. Alle diese kristallographischen Phasen gehen bei Erhitzung des Aluminiumoxids auf Temperaturen über 1100°C in das thermisch stabile α-Aluminiumoxid über. Aktives Aluminiumoxid wird kommerziell für katalytische Anwendungen in verschiedenen Qualitäten und Lieferformen angeboten.

Besonders geeignet für die Herstellung von Trägerkatalysatoren sind Formkörper aus granuliertem oder stranggepresstem Aluminiumoxid mit Korndurchmessern von 1 bis 5 mm, einer spezifischen Oberfläche von 180 bis 400 m²/g, einem Gesamtporenvolumen zwischen 0,3 und 1,2 ml/g sowie einer Schüttdichte von 300 bis 900 g/1. Für die Zwecke der Erfindung wird bevorzugt Aluminiumoxid mit mehr als 200 m²/g spezifischer Oberfläche verwendet, da die katalytische Aktivität des fertigen Katalysators mit steigender Oberfläche des Aluminiumoxids leicht ansteigt. Dieses Material wird in Pulverform bevorzugt für die Herstellung der Schalenkatalysatoren, Extrudate oder Preßlinge eingesetzt.

Das Trägermaterial wird erfindungsgemäss im allgemeinen nicht mit Halogenwasserstoffsäure vorbehandelt.

Die wässrige Imprägnierlösung zur Aufbringung des Promotors kann in einfacher Weise aus in Wasser löslichen Cäsium Wolfram- und Halogenverbindungen, insbesondere Wolframsäure (H₂WO₄), Cäsium hydroxiden, Cäsium halogeniden und gegebenenfalls Ammoniumhalogenide oder Halogenwasserstoffsäure hergestellt werden. Hierzu wird z. B. Wolframsäure in Wasser suspendiert und unter Zugabe einer Base und Erwärmen aufgelöst. Das oder die genannten Alkalihalogenid(e)oder Ammoniumhalogenide, gegebenenfalls auch die entsprechenden Hydroxide und/oder beispielsweise eine Halogenwasserstoffsäure mit dem gewünschten Halogenid werden ebenfalls in Wasser aufgelöst und mit der Lösung der Wolframsäure so vereinigt (Promotorlösung), dass sich die gewünschte Zusammensetzungsverhältnisse für die Cäsium wolframate und ihren Halogenidgehalt ergeben. Vorteilhaft einsetzbar sind neben den Cäsium halogeniden auch Cäsium salze, deren Anionen sich durch Wärmebehandlung rückstandslos austreiben lassen wie z.B. Nitrate, Formiate, Oxalate, Acetate oder Carbonate. Zur Stabilisierung der Promotorlösung mit einem pH von bevorzugt 8 bis 14 werden anorganische und auch organische Basen eingesetzt. Bevorzugt werden solche verwendet, die sich durch eine abschließende Wärmebehandlung des nach der Imprägnierung erhaltenen Katalysators rückstandslos austreiben lassen. Zu diesen Basen gehören bevorzugt Ammoniumhydroxid und organische Basen, insbesondere Amine.

Durch dieses Vorgehen werden die an der Oberfläche von zum Beispiel Al₂O₃-Trägermaterialien vorhandenen sauren Gruppen weitgehend, im allgemeinen mindestens etwa 75 %, im besonderen 100 % neutralisiert.

Das molare Verhältnis von Alkalimetallverbindungen und Halogeniden in der wässrigen Imprägnierlösung derart gewählt, dass die neuen Wolframate Halogenide und Cäsium in einem molaren Verhältnis von 0,01:1 bis 3:1 enthalten. Dies führt im Vergleich zu den bekannten halogenidfreien Katalysatoren zu einer deutlich erhöhten Ausbeute bei Verwendung der erfindungsgemäßen Katalysatoren, insbesondere bei niedrigen Verhältnissen von Schwefelwasserstoff und Methanol im Reaktionsgas.

Als Halogenide werden Fluorid, Bromid und Chlorid bevorzugt, insbesondere Fluorid und Bromid.

Wolframate mit Gehalten von verschiedenen Halogeniden enthalte Kationen von Cäsium und mindestens einem Halogenid in einem Verhältnis von Cäsium zu Halogenid zwischen 0,01:1,0 und 3,0:1,0, wobei die molaren Anteile von gegebenenfalls vorhandenen unterschiedlichen Halogeniden als Summe gezählt werden. Dabei wird der Anteil des kostengünstigeren Halogenids so weit gesteigert und gleichzeitig derjenige des vergleichsweise teureren Halogenids im Gegenzug verringert, dass keine Verschlechterung der Aktivität oder Selektivität des Katalysators eintritt.

Für das Aufbringen der Promotorlösung können verschiedene Imprägniertechniken wie das Tauchimprägnieren, Sprühimprägnieren, Vakuumimprägnieren und die Porenvolumenimprägnierung eingesetzt werden, wobei die Imprägnierung auch mehrfach erfolgen kann. Bei Formkörpern muss das gewählte Imprägnierverfahren es ermöglichen, die gewünschte Beladungsmenge des Promotors mit guter Gleichmäßigkeit über den gesamten Querschnitt aufzubringen.

Bevorzugt wird die Promotorlösung durch Sprüh- oder Vakuumimprägnierung in einem oder in zwei Schritten auf die Formkörper aufgebracht. Bei der Sprühimprägnierung wird die wässrige Imprägnierlösung auf die Trägerkörper gesprüht. Bei der Vakuumimprägnierung wird in einem mit den Formkörpern gefüllten Behälter mittels einer Vakuumpumpe ein Unterdruck erzeugt. Durch Öffnen einer Schlauchverbindung zur wässrigen Imprägnierlösung wird die Lösung in den Behälter gesaugt, bis die gesamte Schüttung der Formkörper mit der Lösung bedeckt ist. Nach einer Imprägnierzeit von 0,2 bis 2 Stunden wird die nicht durch das Material aufgenommene Lösung abgelassen oder abgegossen.

Durch Vortrocknung für die Dauer von 1 bis 10 Stunden bei Raumtemperatur kann sich das anfängliche Konzentrationsgefälle über den Querschnitt der Formkörper weitgehend ausgleichen. Somit wird die Gleichmäßigkeit der Imprägnierung über den Querschnitt der Katalysatorpartikel verbessert. Bevorzugt werden die so erhaltenen Katalysatorvorstufen für die Dauer von 1 bis 10 Stunden bei 100 bis 200, bevorzugt 100 bis 140°C zur Entfernung der Restfeuchte getrocknet. Dann erfolgt für die Dauer von 1 bis 20, bevorzugt 1 bis 5 Stunden eine Kalzinierung bei 300 bis 600, bevorzugt 420 bis 480°C. Dadurch wird der Promotor auf dem Aluminiumoxid fixiert und die Base der Imprägnierlösung zersetzt und ausgetrieben. Optional kann die Schüttung der Trägerkörper der Katalysatorvorstufen bei der Vortrocknung, Trocknung und Kalzinierung von einem Gasstrom durchströmt werden, was den Abtransport der Restfeuchte und der Zersetzungsgase verbessert.

Die Imprägnierung der Formkörper kann auch mehrstufig, insbesondere zweistufig erfolgen.

In dieser Ausführungsform enthält dann die in der ersten Stufe eingesetzte Lösung ein bis zu zwei Drittel der vorgesehenen Gesamtmenge an Cäsium- und Wolframverbindungen.

Geht man mehrstufig, mindestens aber zweistufig vor, kalziniert man die im ersten Schritt erhaltene Vorstufe gegebenenfalls nicht.

Ansonsten läuft in der zweiten Stufe dasselbe Imprägnier-, Trocknungs- und Kalzinierungsprogramm wie für das einstufige Verfahren beschrieben ab.

Diese mehrstufige Imprägnierung ist vor allem dann sinnvoll, wenn hohe Beladungen gewünscht werden und/oder die begrenzte Löslichkeit der Promotormischung die Beladung in einem Schritt nicht ermöglichen.

Es besteht auch die Möglichkeit, die Trägerkörper bzw. das Trägermaterial während des Imprägniervorgangs mehrfach mit der Imprägnierlösung zu besprühen und zwischen diesen Behandlungsschritten jeweils Teile der Restfeuchte bei einer Temperatur von bis zu 120°C zu entfernen, bevor man zu Schritt b übergeht.

Bei der Herstellung des Schalenkatalysators kann das zur Bildung einer Schale aufzubringende Pulver vor oder nach der Beschichtung kalziniert werden. Beispielsweise kann dieser Katalysatortyp gemäß EP-B-0 068 193 hergestellt werden. Auch bei der Herstellung der Extrudate oder der Preßlinge kann die Kalzinierung vor und/oder nach der Verformung erfolgen.

### Beispiele

### Beispiel 1 (Vergleichsbeispiel)

150 g Aluminiumoxid (Spheralite 501A) wurde mit 21,0 Gew.-% Cäsiumwolframat (Cs_{2,0}WO₄) mit Hilfe der Vakuumimprägnierung imprägniert. Hierzu wurde im einzelnen wie folgt vorgegangen:
Zur Herstellung der Imprägnierlösung wurden 55,7 g Wolframsäure in 44,5 g Wasser suspendiert und durch Zugabe von 111,4 g 25 %-iger Ammoniaklösung und Erwärmen auf 50°C gelöst. 74,6 g Cs(OH)·H₂O wurden in 37,3 g Wasser gelöst und mit der ersten Lösung vermischt. Die Lösung wurde anschließend im abgedeckten Becherglas 48 Stunden gerührt.
Daraufhin wurde die Lösung mit 25 g Wasser auf ein Volumen von 234 ml aufgefüllt.

Das Aluminiumoxid wurde in einem Glasgefäß, das auf 150 mbar evakuiert wurde, vorgelegt. Durch das Öffnen eines Hahns wurde die Imprägnierlösung so lange in das evakuierte Glasgefäß gesaugt bis die gesamte Schüttung der Formkörper mit der Lösung bedeckt war. Nach einer Wartezeit von 15 Minuten und Belüftung des Glasgefäßes floss die nicht durch das Aluminiumoxid aufgenommene Lösung zurück in das Becherglas. Vom Aluminiumoxid wurden dabei 79 ml Imprägnierlösung aufgenommen.

Das Granulat wurde für die Dauer von 1 Stunde bei Raumtemperatur im Luftstrom und anschließend bei 120°C für 3 Stunden zur Entfernung der Restfeuchte getrocknet. Danach wurde das Granulat 3 Stunden lang bei 455°C kalziniert.

### Beispiel 2 (Vergleichsbeispiel)

Vergleichsbeispiel 1 wurde mit 26,3 %-iger Beladung des Aluminiumoxids mit Cäsiumwolframat (Cs_{2,0}WO₄) wiederholt.

### Beispiel 3 (Vergleichsbeispiel)

Vergleichsbeispiel 1 wurde mit 19,6 %-iger Beladung des Aluminiumoxids mit Kaliumwolframat (K_{2,0}WO₄) unter Verwendung von KOH statt Cs(OH) · H₂O wiederholt.

### Beispiel 4

150 g Aluminiumoxid (Spheralite 501A) wurde mit 22,3 Gew.-% fluoridhaltigem Cäsiumwolframat mit Hilfe der Vakuumimprägnierung imprägniert. Hierzu wurde im einzelnen wie folgt vorgegangen:
Zur Herstellung der Imprägnierlösung wurden 56,8 g Wolframsäure in 45,4 g Wasser suspendiert und durch Zugabe von 113,6 g 25 %-iger Ammoniaklösung und Erwärmen auf 50°C gelöst. 68,8 g CsF wurden in 40,0 g Wasser gelöst und mit der ersten Lösung vermischt. Die Lösung wurde anschließend im abgedeckten Becherglas 4 Stunden gerührt. Daraufhin wurde die Lösung mit 24,9 g Wasser auf ein Volumen von 234 ml aufgefüllt.

Das Aluminiumoxid wurde in einem Glasgefäß, das auf 150 mbar evakuiert wurde, vorgelegt. Durch das Öffnen eines Hahns wurde die Imprägnierlösung so lange in das evakuierte Glasgefäß gesaugt bis die gesamte Schüttung der Formkörper mit der Lösung bedeckt war. Nach einer Wartezeit von 15 Minuten und Belüftung des Glasgefäßes floss die nicht durch das Aluminiumoxid aufgenommene Lösung zurück in das Becherglas. Vom Aluminiumoxid wurden dabei 73 ml Imprägnierlösung aufgenommen.

Das Granulat wurde für die Dauer von 1 Stunde bei Raumtemperatur im Luftstrom und anschließend bei 120°C für 3 Stunden zur Entfernung der Restfeuchte getrocknet. Danach wurde das Granulat 3 Stunden lang bei 455°C kalziniert.

### Beispiel 5

150 g Aluminiumoxid (Spheralite 501A) wurden in einer Imprägnierung mit insgesamt 23,9 Gew.-% chloridhaltigem Cäsiumwolframat mit Hilfe der Vakuumimprägnierung imprägniert. Im einzelnen wurde wie folgt vorgegangen:
57,8 g Wolframsäure wurden in 46,2 g Wasser suspendiert und durch Zugabe von 115,6 g 25 %-iger Ammoniaklösung und Erwärmen auf 50°C gelöst. 77,6 g CsCl wurden in 30,0 g Wasser gelöst und mit der ersten Lösung vermischt. Die Lösung wurde anschließend im abgedeckten Becherglas 22 Stunden gerührt. Daraufhin wurde die Lösung mit 23,2 g Wasser auf ein Volumen von 234 ml aufgefüllt. Das Aluminiumoxid wurde in einem Glasgefäß, das auf 150 mbar evakuiert wurde, vorgelegt. Durch das Öffnen eines Hahns wurde die Imprägnierlösung so lange angesaugt bis die gesamte Schüttung der Formkörper mit der Lösung bedeckt war. Nach einer Wartezeit von 15 Minuten und Belüftung des Glasgefäßes floss die nicht durch das Aluminiumoxid aufgenommene Lösung zurück in das Becherglas. Vom Aluminiumoxid wurden dabei 74 ml Imprägnierlösung aufgenommen. Anschließend wurde das Granulat 1 Stunde bei Raumtemperatur und 3 Stunden bei 120°C getrocknet sowie 3 Stunden bei 455°C kalziniert.

### Beispiel 6

150 g Aluminiumoxid (Spheralite 501A) wurden in einer zweistufigen Imprägnierung mit insgesamt 18,5 Gew.-% bromidhaltigem Cäsiumwolframat mit Hilfe der Vakuumimprägnierung imprägniert. Im einzelnen wurde wie folgt vorgegangen:
58,5 g Wolframsäure wurden in 46,8 g Wasser suspendiert und durch Zugabe von 116,9 g 25 %-iger Ammoniaklösung und Erwärmen auf 50°C gelöst. 15,6 g CsBr und 50,4 g Cs(OH)·H₂O wurden in 30,0 g Wasser gelöst und mit der ersten Lösung vermischt. Die Lösung wurde anschließend im abgedeckten Becherglas 21 Stunden gerührt. Daraufhin wurde die Lösung mit 17,2 g Wasser auf ein Volumen von 234 ml aufgefüllt. Das Aluminiumoxid wurde in einem Glasgefäß, das auf 150 mbar evakuiert wurde, vorgelegt. Durch das Öffnen eines Hahns wurde die Imprägnierlösung so lange angesaugt bis die gesamte Schüttung der Formkörper mit der Lösung bedeckt war. Nach einer Wartezeit von 15 Minuten und Belüftung des Glasgefäßes floss die nicht durch das Aluminiumoxid aufgenommene Lösung zurück in das Becherglas. Vom Aluminiumoxid wurden dabei 81 ml Imprägnierlösung aufgenommen. Anschließend wurde das Granulat 1 Stunde bei Raumtemperatur und 3 Stunden bei 120°C getrocknet sowie 3 Stunden bei 455°C kalziniert.

### Beispiel 7

150 g Aluminiumoxid (Spheralite 501A) wurden in einer Imprägnierung mit insgesamt 29,6 Gew.-% jodidhaltigem Cäsiumwolframat mit Hilfe der Vakuumimprägnierung imprägniert. Im einzelnen wurde wie folgt vorgegangen:
64,1 g Wolframsäure wurden in 51,3 g Wasser suspendiert und durch Zugabe von 128,2 g 25 %-iger Ammoniaklösung und Erwärmen auf 50°C gelöst. 132,7 g CsJ wurden in 30,0 g Wasser gelöst und mit der ersten Lösung vermischt. Die Lösung wurde anschließend im abgedeckten Becherglas 6 Stunden gerührt. Daraufhin wurde die Lösung mit 6 g Wasser auf ein Volumen von 234 ml aufgefüllt. Das Aluminiumoxid wurde in einem Glasgefäß, das auf 150 mbar evakuiert wurde, vorgelegt. Durch das Öffnen eines Hahns wurde die Imprägnierlösung so lange angesaugt bis die gesamte Schüttung der Formkörper mit der Lösung bedeckt war. Nach einer Wartezeit von 15 Minuten und Belüftung des Glasgefäßes floss die nicht durch das Aluminiumoxid aufgenommene Lösung zurück in das Becherglas. Vom Aluminiumoxid wurden dabei 80 ml Imprägnierlösung aufgenommen. Anschließend wurde das Granulat 1 Stunde bei Raumtemperatur und 3 Stunden bei 120°C getrocknet sowie 3 Stunden bei 455°C kalziniert.

### Beispiel 8

95 g Aluminiumoxid (Spheralite 501A) wurden in einer Imprägnierung mit insgesamt 23,5 Gew.-% fluorid- und bromidhaltigem Cäsiumwolframat mit Hilfe der Vakuumimprägnierung imprägniert. Im einzelnen wurde wie folgt vorgegangen:
36,8 g Wolframsäure wurden in 35,0 g Wasser suspendiert und durch Zugabe von 73,6 g 25 %-iger Ammoniaklösung und Erwärmen auf 50°C gelöst. 22,3 g CsF und 31,2 g CsBr wurden in 30,0 g Wasser gelöst und mit der ersten Lösung vermischt. Die Lösung wurde anschließend im abgedeckten Becherglas 22 Stunden gerührt. Daraufhin wurde die Lösung mit 1 g Wasser auf ein Volumen von 234 ml aufgefüllt. Das Aluminiumoxid wurde in einem Glasgefäß, das auf 150 mbar evakuiert wurde, vorgelegt. Durch das Öffnen eines Hahns wurde die Imprägnierlösung so lange angesaugt bis die gesamte Schüttung der Formkörper mit der Lösung bedeckt war. Nach einer Wartezeit von 15 Minuten und Belüftung des Glasgefäßes floss die nicht durch das Aluminiumoxid aufgenommene Lösung zurück in das Becherglas. Vom Aluminiumoxid wurden dabei 44 ml Imprägnierlösung aufgenommen. Anschließend wurde das Granulat 1 Stunde bei Raumtemperatur und 3 Stunden bei 120°C getrocknet sowie 3 Stunden bei 455°C kalziniert.

### Beispiel 9 (Anwendungsbeispiel)

Die Katalysatoren wurden bezüglich ihrer Leistungsdaten bei der Synthese von Methylmercaptan aus Schwefelwasserstoff und Methanol getestet.

Die Synthese wurde in einem Edelstahlrohr von 18 mm Innendurchmesser und einer Länge von 500 mm durchgeführt. Die Katalysatorschüttung von jeweils 76 ml wurde beiderseits durch Inertschüttungen aus Glaskugeln im Reaktionsrohr fixiert. Das Reaktionsrohr wurde über einen Doppelmantel mit einem Thermoöl auf die Reaktionstemperatur von etwa 320°C aufgeheizt.

Die Versuchsbedingungen sind der folgenden Aufstellung zu entnehmen:

| | |
|---|---|
| GHSV: | 1300 h⁻¹ (bezogen auf Normbedingungen) |
| LHSV: | 0,84 h⁻¹ (bezogen auf flüssiges MeOH) |
| Reaktionstemperatur: | 320°C |
| Massenverhältnis | |
| H₂S/MeOH: | 1,9 |
| Druck: | 9 bar |

Das Reaktionsgemisch mit den Produkten Methylmercaptan, Dimethylsulfid und Dimethylether und mit den nicht umgesetzten Eingangsstoffen Methanol und Schwefelwasserstoff wird durch online Gaschromatographie analysiert.

Werden dem Katalysator Halogenide zugesetzt, so ist eine deutlich Steigerung der Aktivität bei gleichzeitig verbesserter Selektivität zu erkennen. Während im Stand der Technik die Selektivität speziell bei hohen Umsatzgraden sinkt, werden durch den Zusatz der Halogene auch bei sehr hohen Umsätzen noch unverändert hohe Selektivitäten festgestellt. Dies führt im Vergleich zum Stand der Technik zu einer Ausbeutesteigerung von bis zu 15 %. Die Selektivität kann durch Einstellung des Cäsium-Wolframat-Halogenid-Verhältnisses auf bis - 96,6 % gesteigert werden. Dies führt bei der großtechnischen Synthese von Methylmercaptan zu erheblichen Kosteneinsparungen bei der Abtrennung des Reaktionsproduktes von nicht umgesetztem Methanol und Nebenprodukten.

Weiterhin zeigen die Ergebnisse der Beispiele, dass zumindest ein Teil der Halogenide gegeneinander ausgetauscht werden kann, um die Aktivität und Selektivität des Katalysators gezielt einzustellen oder um Rohstoffkosten bei der Katalysatorfertigung einzusparen.

**Tabelle 1: Versuchsergebnisse**

| Katalysator | Mol.Verhält. | Halogen | mol-Verhält. | Beladung | Methanol- | Selek- | Aus- |
|---|---|---|---|---|---|---|---|
| Beispiel | Alkali:W | | Halogen:Alkali | [Gew.-%] | umsatz | tivität | beute |
| | | | | | [%] | [%] | [%] |
| VB1 | 2:1 | - | 0 | 21,0 | 82,4 | 93,3 | 76,9 |
| VB2 | 2:1 | - | 0 | 26,3 | 79,5 | 94,7 | 75,2 |
| VB3 | 2:1 | - | 0 | 19, 6 | 76,0 | 95,2 | 72,4 |
| B4 | 2:1 | F | 1:1 | 22,3 | 75,4 | 96,6 | 72,8 |
| B5 | 2:1 | Cl | 1:1 | 23,9 | 95,0 | 93,7 | 89,0 |
| B6 | 1,6:1 | Br | 0,2:1 | 18,5 | 92,4 | 94,5 | 87,3 |
| B7 | 2:1 | J | 1:1 | 29,6 | 88,9 | 95,5 | 84,9 |
| B8 | 2:1 | F, Br | 1:1 | 23,5 | 91,4 | 96,6 | 88,3 |

| | | | | | | | |
|---|---|---|---|---|---|---|---|
| VB1: Katalysator nach Vergleichsbeispiel 1 | | | | | | | |

## Patentansprüche

1. Alkaliwolframat enthaltende Katalysatoren, wobei das Alkaliwolframat mindestens ein Halogenid enthält und das im Wolframat gebundene Alkalimetall Cäsium ist.

2. Katalysator gemäss Anspruch 1, wobei das Wolframat ein Halogenid, ausgewählt aus der Gruppe F, Br, J, C1 enthält.

3. Katalysator gemäss Anspruch 1, wobei das Wolframat zwei Halogenide, ausgewählt aus der Gruppe F, C1, Br, J, enthält.

4. Katalysator gemäss den Ansprüchen 1 bis 3, wobei das Wolframat Cäsium als gebundenes Alkalimetall, Wolfram und Halogenid(e) in einem molaren Verhältnis von Cäsium zu Wolfram von 0,9 bis < 4:1 und in einem molaren Verhältnis der Summe der Halogenide zu Cäsium von 0,01 bis 3:1 enthält.

5. Katalysator gemäß Anspruch 4, **dadurch gekennzeichnet, dass** sich das molare Verhältnis von Cäsium zu Wolfram im halogenidhaltigen Cäsiumwolframat auf 2,2:1 bis 0,9:1 und das molare Verhältnis von Halogenid(en) zu Alkalimetall sich auf 1:1 bis 0,01:1 beläuft.

6. Katalysator gemäss den Ansprüchen 1 bis 4, **gekennzeichnet durch** ein Trägermaterial und das halogenidhaltige Cäsiumwolframat.

7. Katalysator gemäss Anspruch 6, **gekennzeichnet durch** ein Halogenwasserstoffsäure-freies Trägermaterial.

8. Katalysator gemäss den Ansprüchen 6 und 7, bestehend aus einem Schalenkatalysator, bei dem ein Kern als Trägermaterial mit dem halogenidhaltigen Cäsiumwolframat oder mit einem mit diesem halogenidhaltigen Cäsiumwolframat imprägnierten Trägermaterial umhüllt ist.

9. Katalysator gemäss den Ansprüchen 6 und 7, bei dem das mit dem halogenidhaltigem Cäsiumwolframat imprägnierte Trägermaterial als Vollkatalysator vorliegt.

10. Katalysator gemäss Anspruch 7, bei dem die Oberfläche eines geformten Trägermaterials mit Cäsiumwolframat und Halogeniden mit einem molaren Verhältnis von Cäsium zu Wolfram 0,9 bis < 4:1 und einem molaren Verhältnis von Halogenid(en) zu Cäsium von 0,01 bis 3:1 imprägniert ist.

11. Katalysator gemäss den Ansprüchen 1 bis 10, **dadurch gekennzeichnet, dass** das Wolframat der allgemeinen Formel
AₓWO_{y}X_{z} (I)
entspricht, in der bedeuten
A: Cäsium
X : Halogenid(e)
x:0,9 bis < 4
y: Sauerstoffgehalt im Oxid
z:0,01 bis <12.

12. Katalysator gemäss einem oder mehreren der Ansprüche 6 bis 11, **dadurch gekennzeichnet, dass** er das halogenidhaltige Cäsiumwolframat in einer Menge von 8 bis 50 Gew.-%, bevorzugt 20 bis 36 Gew.-%, enthält.

13. Katalysator gemäss einem oder mehreren der Ansprüche 6 bis 11, **dadurch gekennzeichnet, dass** der Trägerkörper oder das Trägermaterial aus einer oxidischen anorganischen Verbindung besteht.

14. Katalysator gemäss Anspruch 13, **dadurch gekennzeichnet, dass** der Trägerkörper oder das Trägermaterial aus Aluminiumoxid (Al₂O₃) besteht.

15. Katalysator gemäss Anspruch 14, **dadurch gekennzeichnet, dass** das Trägermaterial eine spezifische Oberfläche von 180 bis 400 m²/g (BET) und ein Gesamtporenvolumen von 0,3 bis 1,2 ml/g, besitzt.

16. Verfahren zur Herstellung von Alkylmercaptanen durch Umsetzung von Alkanolen und Schwefelwasserstoff in Gegenwart des Katalysators gemäss den Ansprüchen 1 bis 15.

17. Verfahren gemäss Anspruch 16 zur Herstellung von Methylmercaptan durch Umsetzung von Methylalkohol und Schwefelwasserstoff.

## Claims

1. Catalyst comprising alkali metal tungstate, wherein the alkali metal tungstate contains at least one halide and the alkali metal bound within the tungstate is caesium.

2. Catalyst according to Claim 1, wherein the tungstate contains a halide selected from the group consisting of F, Br, I and Cl.

3. Catalyst according to Claim 1, wherein the tungstate contains two halides selected from the group consisting of F, Cl, Br and I.

4. Catalyst according to Claims 1 to 3, wherein the tungstate contains caesium as the bound alkali metal, tungsten and halide(s) in a molar ratio of caesium to tungsten of 0.9 to < 4:1 and in a molar ratio of the sum of the halides to caesium of 0.01 to 3:1.

5. Catalyst according to Claim 4, **characterized in that** the molar ratio of caesium to tungsten in the halide-containing caesium tungstate is from 2.2:1 to 0.9:1, and the molar ratio of halide(s) to alkali metal is from 1 to 0.01:1.

6. Catalyst according to Claims 1 to 4, **characterized by** a support material and the halide-containing caesium tungstate.

7. Catalyst according to Claim 6, **characterized by** a hydrohalic acid-free support material.

8. Catalyst according to Claims 6 and 7, consisting of an eggshell catalyst in which a core as the support material is coated with the halide-containing caesium tungstate or with a support material impregnated with this halide-containing caesium tungstate.

9. Catalyst according to Claims 6 and 7, in which the support material impregnated with the halide-containing caesium tungstate is in the form of an unsupported catalyst.

10. Catalyst according to Claim 7, in which the surface of a shaped support material is impregnated with caesium tungstate and halides with a molar ratio of caesium to tungsten of 0.9 to < 4:1 and a molar ratio of halide(s) to caesium of 0.01 to 3:1.

11. Catalyst according to Claims 1 to 10, **characterized in that** the tungstate corresponds to the general formula
AₓWO_{y}X_{z} (I)
in which
A: caesium
X: halide(s)
x: 0.9 to < 4
y: oxygen content in the oxide
z: 0.01 to <12.

12. Catalyst according to one or more of Claims 6 to 11, **characterized in that** it contains the halide-containing caesium tungstate in an amount of 8 to 50% by weight, preferably 20 to 36% by weight.

13. Catalyst according to one or more of Claims 6 to 11, **characterized in that** the support body or the support material consists of an oxidic inorganic compound.

14. Catalyst according to Claim 13, **characterized in that** the support body or the support material consists of aluminium oxide (Al₂O₃) .

15. Catalyst according to Claim 14, **characterized in that** the support material has a specific surface area of 180 to 400 m²/g (BET) and a total pore volume of 0.3 to 1.2 ml/g.

16. Process for preparing alkyl mercaptans by converting alkanols and hydrogen sulphide in the presence of the catalyst according to Claims 1 to 15.

17. Process according to Claim 16 for preparing methyl mercaptan by reacting methyl alcohol and hydrogen sulphide.

## Revendications

1. Catalyseurs contenant un tungstate de métal alcalin, dans lesquels le tungstate de métal alcalin contient au moins un halogénure et le métal alcalin lié dans le tungstate est le césium.

2. Catalyseur selon la revendication 1, dans lequel le tungstate contient un halogénure choisi dans le groupe constitué par F, Br, I, Cl.

3. Catalyseur selon la revendication 1, dans lequel le tungstate contient deux halogénures choisis dans le groupe constitué par F, Cl, Br, I.

4. Catalyseur selon les revendications 1 à 3, dans lequel le tungstate contient du césium en tant que métal alcalin lié, du tungstène et un/des halogénure(s) en un rapport molaire du césium au tungstène de 0,9 à < 4:1 et en un rapport molaire de la somme des halogénures au césium de 0,01 à 3:1.

5. Catalyseur selon la revendication 4, **caractérisé en ce que** le rapport molaire du césium au tungstène dans le tungstate de césium contenant un/des halogénure(s) va de 2,2:1 1 à 0,9:1 1 et le rapport molaire de l'halogénure/des halogénures au métal alcalin va de 1:1 à 0,01:1.

6. Catalyseur selon les revendications 1 à 4, **caractérisé par** une matière de support et le tungstate de césium contenant un/des halogénure(s).

7. Catalyseur selon la revendication 6, **caractérisé par** une matière de support exempte d'hydracide halogéné.

8. Catalyseur selon les revendications 6 et 7, consistant en un catalyseur à enveloppe, dans lequel un noyau en tant que matière de support est enrobé avec le tungstate de césium contenant un/des halogénure(s) ou avec une matière de support imprégnée de ce tungstate de césium contenant un/des halogénure(s).

9. Catalyseur selon les revendications 6 et 7, dans lequel la matière de support imprégnée du tungstate de césium contenant un/des halogénure(s) est présente en tant que catalyseur massif.

10. Catalyseur selon la revendication 7, dans lequel la surface d'une matière de support moulée est imprégnée avec du tungstate de césium et des halogénures en un rapport molaire du césium au tungstène de 0,9 à < 4,1 et un rapport molaire de l'halogénure/des halogénures au césium de 0,01 à 3:1.

11. Catalyseur selon les revendications 1 à 10, **caractérisé en ce que** le tungstate correspond à la formule générale
AₓWO_{y}X_{z} (I)
, dans laquelle
A représente le césium
X représente un/des halogénure(s)
x vaut de 0,9 à < 4
Y représente la teneur en oxygène de l'oxyde
z vaut de 0,01 à < 12.

12. Catalyseur selon une ou plusieurs des revendications 6 à 11, **caractérisé en ce qu'**il contient le tungstate de césium contenant un/des halogénure(s) en une quantité de 8 à 50 % en poids, de préférence de 20 à 36 % en poids.

13. Catalyseur selon une ou plusieurs des revendications 6 à 11, **caractérisé en ce que** le corps de support ou la matière de support consiste en un composé inorganique de type oxyde.

14. Catalyseur selon la revendication 13, **caractérisé en ce que** le corps de support ou la matière de support consiste en oxyde d'aluminium (Al₂O₃).

15. Catalyseur selon la revendication 14, **caractérisé en ce que** la matière de support a une surface spécifique de 180 à 400 m²/g (BET) et un volume total de pores de 0,3 à 1,2 ml/g.

16. Procédé pour la préparation d'alkylmercaptans par mise en réaction d'alcanols et d'hydrogène sulfuré en présence du catalyseur selon les revendications 1 à 15.

17. Procédé selon la revendication 16, pour la préparation de méthylmercaptan par mise en réaction d'alcool méthylique et d'hydrogène sulfuré.
